# EUROPEAN PATENT APPLICATION

(11) **EP 1 801 222 A1**
(43) Date of publication of application: **27.06.2007**
(21) Application number: 05857312.2
(22) Date of filing: 14.07.2005
(51) Int. Cl.: C12N 15/82, C12N 5/10, A01H 5/00

(54) **PARTHENOCARPIC TOMATOES AND PRODUCTION METHOD THEREOF**

(30) Priority: 17.07.2004 ES 200401761
(71) Applicant: Consejo Superior de Investigaciones Cientificas, 28006 Madrid (ES); NEWBIOTECHNIC, S.A., 41110 Bollullos de la Mitacion (Sevilla) (ES)
(72) Inventor: ROQUE MESA, Edelín M., E-46022 Valencia (ES); ELLUL, Phillipe, E-46022 Valencia (ES); GÓMEZ JIM NEZ, María, Dolores, E-46022 Valencia (ES); MADUEÑO ALBI, Francisco, E-46022 Valencia (ES); BELTRAN PORTER, José, Pío, E-46022 Valencia (ES); CAÑAS CLEMENTE, Luis, Antonio, E-46022 Valencia (ES)
(74) Representative: Ungria Lopez, Javier
(86) International application number: PCT/ES2005/070102
(87) International publication number: WO 2006/095034

(57) **Abstract**

The invention relates to parthenocarpic tomatoes (*Lycopersicon* esculentum *Mill.)* which are grown from seeds, which are smaller than wild tomatoes and which, when ripe, display a more intense red colour than wild tomatoes. The tomatoes are obtained by cultivating a transgenic tomato plant which produces said parthenocarpic tomatoes and which is obtained using a method consisting in: (a) introducing a DNA construction into a cell or tissue of a tomato plant, said construction comprising (i) the pea *END1* gene promoter or a functional fragment of same and (ii) a cytotoxic gene which is functionally bound to the aforementioned promoter or fragment of same; and (b) regenerating the tomato plant cell or tissue transformed in step (a) in order to produce a transgenic tomato plant that produces parthenocarpic tomatoes. The invention is suitable for use in food and agrifood industries.

## Description

### FIELD OF THE INVENTION

The invention relates to parthenocarpic tomato fruits (*Lycopersicon esculentum* Mill.) and a method for their production. The invention also relates to a method for producing a transgenic tomato plant which produces parthenocarpic tomatoes.

### STATE OF THE ART

### Tomatoes and parthenocarpy

The tomato (*Lycopersicon esculentum* Mill.) is one of the most important crops at world level, with millions of tons being produced each year. The economic importance of this crop requires a continual effort to improve cultivated varieties.

Tomatoes are obtained by harvesting fruits produced by self-pollination of their flowers. For the pollination and fertilisation of flowers to be successful, plants have to grow within a temperature range lying between 15°C and 21°C by night and between 30°C and 35°C by day. Under different climatic conditions, fruits fail to form owing to insufficient and/or incorrect pollen development.

The fruit of the wild tomato contains seeds. Although the appearance of seeds in tomato is not usually an important problem *per se* for consumers, it has been described that seedless tomato fruits have more flavour, they have a higher content of dry matter, sugars and soluble solids and less acidity and cellulose than fruits with seeds [Lukyanenko, A.N. (1991). Parthenocarpy in Tomato. In Genetic Improvement of Tomato. Monographs on Theoretical and Applied Genetics. 14. pp. 167-178. Ed. G. Kalloo, Springer-Verlag, ISBN 3-540-53062-2]. Moreover, in processes for preparing foods derived from tomato at industrial scale, such as for example purees, soups, juices or sauces, the possibility of starting from seedless fruits would mean that the seeds would not have to be removed by sieving prior to processing the fruit, in the same way as it would facilitate domestic culinary practices, which frequently include seed elimination prior to cooking. So, one of the aims of tomato improvers is the production of seedless tomatoes.

Parthenocarpy is the production of fruits without fertilisation and it permits seedless fruits to be obtained. Parthenocarpy is favoured by certain environmental conditions such as high or low daytime or nocturnal temperatures, a low level of light and high humidity.

Parthenocarpy in tomatoes can occur naturally or it can be artificially induced. Natural parthenocarpy is produced by genetic causes and can be obligatory or optional, in other words, depending on the environmental conditions. Artificially, parthenocarpy can be induced as a result of treatment of the ovary with exogenous agents such as dead pollen extract, or by means of applying growth regulating substances, either natural or artificial. In fact, with the aim of preventing production losses in adverse conditions and in greenhouse cultivation, exogenous applications of growth regulators are frequently used in order to induce the formation of parthenocarpic fruits. Nevertheless, as well as implying an additional cost in agrochemicals and labour associated with the treatments, these practices also lead to the frequent appearance of malformations of the fruits.

Various tomato cultivars have been identified that produce parthenocarpic fruits owing to mutations in parthenocarpic fruit genes or *pat* genes [Baggett, J.R., Kean, D., Mansour, N.S. (1997). Siletz parthenocarpic tomato. HortScience 32., 1299-1300]. As well as producing parthenocarpic fruits, the pat mutant also shows certain pleiotropic effects such as male and female sterility and malformations in the stamens. In the same way, the antisense expression of the gene of the family MADS box *TM8* of tomato, as well as producing morphological alterations and changes in the identity of the floral organs, and male and female sterility, also provoked the development of parthenocarpic fruits [Lifschitz, E., Brodai, L., Hareven, D., Hurwitz, C., Prihadash, A., Pnueli, L., Samach, A., Zamir, D. (1993). Molecular mapping of flower development in tomato. In Molecular Biology of Tomato, (ed.) J. Yoder, pp. 175-184. Technomic, Lancaster PA, USA. ISBN 0877629927]. Likewise, the repression of the expression of *TM29,* a gene also belonging to the MADS box family of tomato, provokes alterations in the stamens and ovaries and is furthermore associated with the production of parthenocarpic fruits and floral reversion [Ampomah-Dwamena, C., Morris, B.A., Sutherland, P., Veit, B., Y Yao, J-L. (2002). Down-Regulation of TM29, a tomato SEPALLATA homolog, causes parthenocarpic fruit development and floral reversion. Plant Physiol. 130 605-617]. The characterisation of the *stamenless* tomato mutant shows that it displays homeotic conversions similar to those shown by class B mutants of Arabidopsis thaliana and of Antirrhinum majus and that the fruits lack seeds [Gómez, P., Jamilenz, M., Capel, J., Zurita, S, Angosto, T. and Lozano, R. (1999). Stamenless, a tomato mutant with homeotic conversions in petal and stamens. Planta. 209, 172-179]. The association of homeotic transformations affecting the stamens with the development of parthenocarpic fruits has also been described in apple [Yao, J-L., Dong, Y-H., Morris, B.A.M., (2001). Parthenocarpic apple fruit production conferred by transposon insertion mutations in a MADS-box transcription factor. PNAS, 30, 1306-1311]. In general, it can be estimated that given the functions of homeotic genes, their biotechnological use directed at interfering with the development of the stamens would be subject to the appearance of other undesired phenotypic effects.

So, the development of cultivars of parthenocarpic (seedless) tomatoes with commercial interest is a permanent objective of tomato improvers. In fact, parthenocarpic tomatoes would be particularly appreciated by consumers who do not like the presence of seeds in the fruits and, moreover, it would also be beneficial for the agrifood industries since it would permit certain other products such as juices, sauces, pastas, purees, soups, etc., to be obtained more efficiently and economically since the seeds would not have to be removed by sieving the tomato pulp prior to processing.

### Promoter of the pea END1 gene

The promoter of the *END1* gene of pea *(Pisum sativum* L.) is a promoter capable of directing the specific expression in anther of early stages of development of the plant as described and highlighted in patent application WO 01/073088.

### SUMMARY OF THE INVENTION

Surprisingly, it has now been found that it is possible to produce parthenocarpic (seedless) fruits starting from transgenic tomato plants obtained by means of regeneration of tomato plant cells or tissues that have been transformed by means of the introduction of a DNA construction which comprises a cytotoxic gene under the control of the pea *END1* gene promoter or a fragment of that promoter which maintains the regulating capacity of the specific expression in anther. Said method is therefore associated with the obtaining of androsterile plants of tomato by means of the specific expression of a cytotoxic gene in the anthers, such as a gene that produces sterility, for example, the barnase gene, under the control of the pea *END1* gene promoter or a functional fragment thereof.

Androsterile tomato plants have been obtained by transformation mediated by Agrobacterium of *Lycopersicon esculentum,* var Micro-Tom with a construction that includes the barnase gene under the control of the pea *END1* gene promoter. Transgenic plants were obtained *(END1::barnase)* which show morphological and functional alterations in the stamens preventing the formation of viable pollen and which are therefore androsterile. Given that tomato plants are autogamous, viz., self-fertilising, such androsterile plants ought not in theory to develop fruits. Nevertheless, all the transgenic tomato plants *END1::barnase* produce fruits. The characterisation of these fruits reveals the absence of seeds showing that their development had taken place by parthenocarpy, an alternative route to the normal setting and development of the fruit, where the ovary grows without the formation of seeds.

More specifically, by means of transformation mediated by Agrobacterium, cells and tissues of tomato plants (*Lycopersicon esculentum*) var. Micro-Tom with a construction that includes the barnase gene under the control of the pea *END1* gene promoter were transformed. 72 primary transgenic shoots (T1) were generated, of which 25 were selected. The ploidy level of the selected plants was assessed by means of techniques associated with flow cytometry. Of the 25 T1 plants, 24 were diploid and just one was tetraploid. The PCR analysis of tissue samples of diploid plants permitted the transgene to be detected in all of them. 14 plants were selected in order to acclimatise them and grow them in a greenhouse and carry out their phenotypic characterisation. The transgenic plants showed a development pattern similar to that of the wild plants. In both cases the flowers developed normally producing five sepals alternating with five petals and two carpels fused to form a pistil. Nevertheless, while the five stamens of the control plants form the characteristic staminal cone around the style, the phenotype of the stamens of the transgenic plants varied from one plant to another. Transformants were observed which showed a slight reduction in the size of the stamens and a small separation between them and there were other plants with severe phenotype which had the stamens atrophied leaving the style exposed. These plants were androsterile, nevertheless, unlike the androsterile plants of Arabidopsis or tobacco produced by this same experimental approach and which are incapable of forming fruits by themselves, in tomato all the *END1::barnase* plants produce fruits. All the fruits that were produced lacked seeds and were therefore parthenocarpic fruits, independently of the severity of the phenotype in the anthers and the presence of the few pollen grains observed in some mild phenotype transformants. Such pollen grains were shown to be non-viable, in other words, they fail to germinate and are therefore incapable of producing fertilisation. The transgenic, parthenocarpic, fruits are of a size somewhat smaller than the corresponding wild fruits. When the transgenic plants are pollinated by hand using pollen from wild plants, fruits are obtained with characteristics similar to those produced by the wild plants.

Therefore, one aspect of the present invention relates to a method for the production of a transgenic tomato plant which produces parthenocarpic tomatoes. This can be done by means of the stable introduction into the genome of the tomato plant of a DNA construction in which a cytotoxic gene is found under the control of the pea *EMD1* gene promoter or a functional fragment thereof which mediates the expression of that cytotoxic gene in the appropriate region of the plant at the appropriate moment of development. In a particular embodiment, that cytotoxic gene is the barnase gene.

In another aspect the invention relates to a method for producing a parthenocarpic tomato which comprises cultivating a transgenic tomato plant which produces parthenocarpic tomatoes obtained according to the method provided by this invention under conditions which permit the flowering and development of the tomato.

Parthenocarpic tomatoes obtained according to the method indicated above constitute an additional aspect of the present invention.

### BRIEF DESCRIPTION OF THE FIGURES

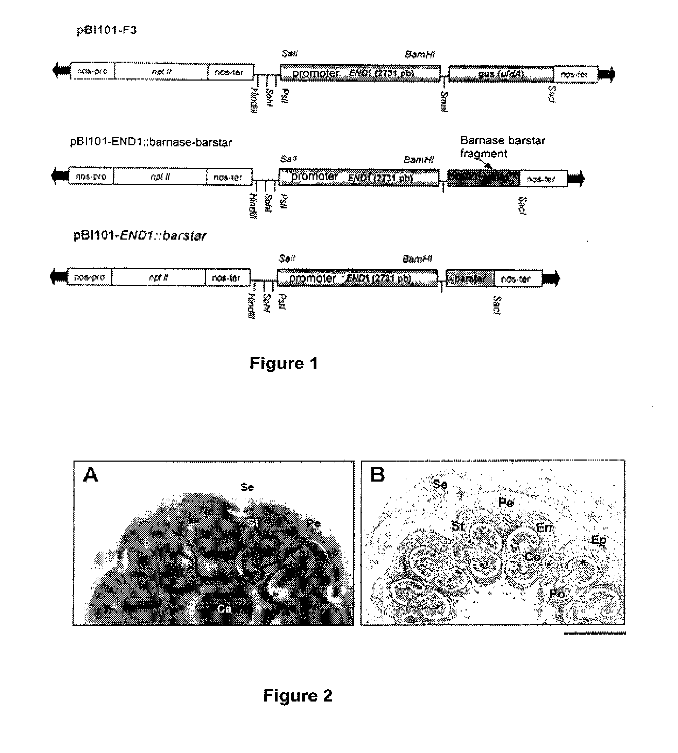

Figure 1 schematically shows various constructions used by the inventors, specifically, the construction used for checking the functionality of the *END1* promoter in plants other than pea by means of expression of a marker gene (GUS) (Figure 1A); the construction used in the production of androsterile transgenic plants of Arabidopsis, tobacco and tomato (Figure 1B); ;and the construction used for the restoration of fertility in androsterile plants of *Arabidopsis* (Figure 1C).
Figure 2 shows the expression of GUS gene in tomato anthers under the direction of the promoter *END1*. Figure 2A: Section of a tomato flower in vivo, showing the expression of GUS in the tissues of the anther involved in the architecture of the pollen sac. Figure 2B: Ditto, but with a section of a flower included in paraffin. Se, sepals, Pe, petals, St, stamens, Ca, carpel, En endothecium, Ep, epidermis, Co, connective, Po, pollen (there is no GUS expression).

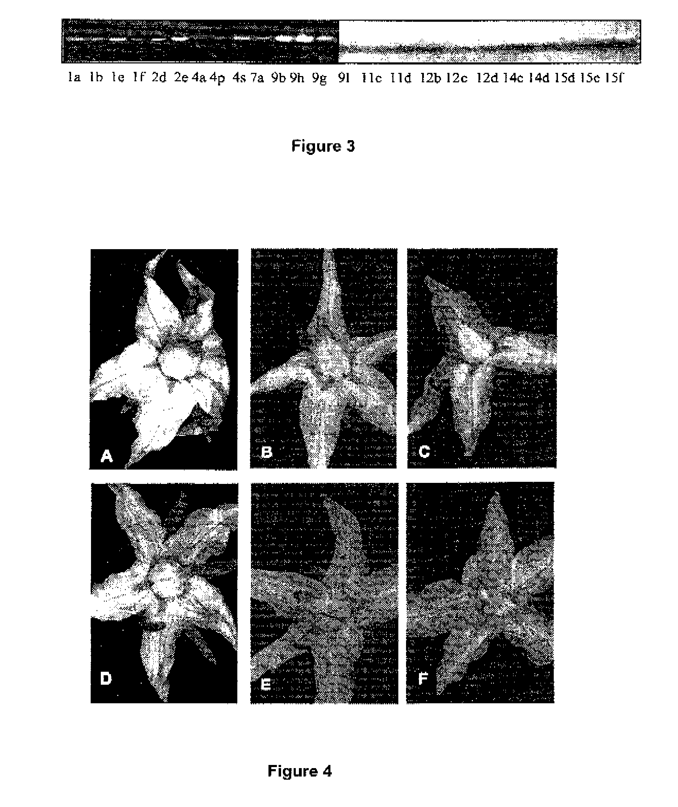

Figure 3 shows the result of PCR amplification of the fragment barnase-barstar of the genome of *END1::barnase* tomato plants (cv. Micro-Tom).
Figure 4: shows the phenotype of flowers of *END1::barnase* transgenic tomato plants. A: Flower of a non-transformed tomato plant; B: Flower of the plant *END1::barnase* 4S, mild malformation phenotype of the anthers; C: Flower of the plant FND1::barnase 14C, mild malformation phenotype of the anthers; D: Flower of the plant *ENDZ::barnase* 9L, medium malformation phenotype of the anthers; E: Flower of the plant *END1::barnase* 12B, medium malformation phenotype of the anthers; F: Flower of the plant *END1::barnase* 1E, severe malformation phenotype of anthers.

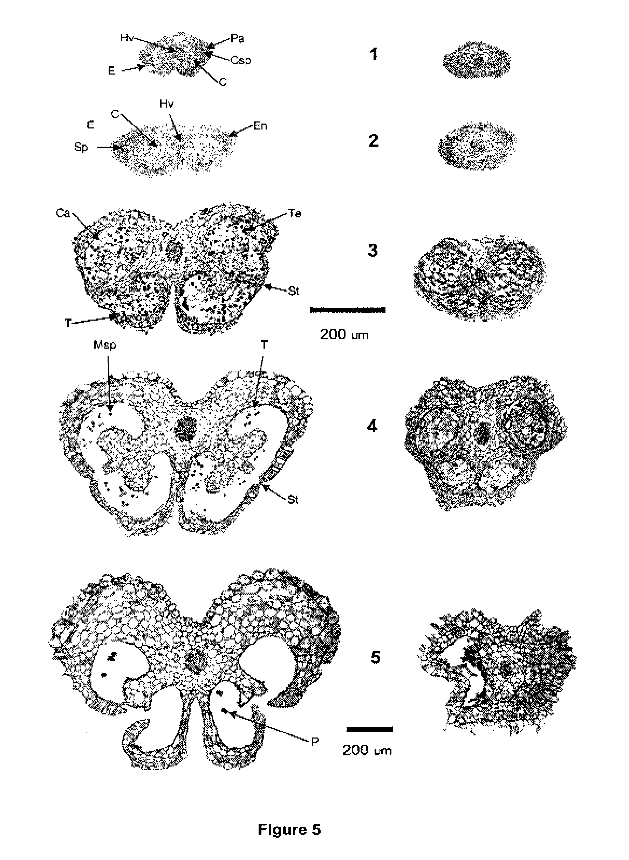

Figure 5 shows the tomato anther development in non-transformed plants and in *END1::barnase* plants with severe malformation phenotype in the anthers. A: Anther development in wild tomato plants (cv. Micro-Tom). B: Anther development in *END1::barnase* tomato plants (cv. Micro-Tom) which showed a severe malformation phenotype of the anthers. E (epidermis), En (endothecium), C (connective), Pa (parietal cells), Csp (sporogen cells); Hv (vascular bundle); Sp (sporogen tissue), Te (tetrads), Ca (callus), T (tapetum), Msp (microspores), St (stomium), P (pollen grains).

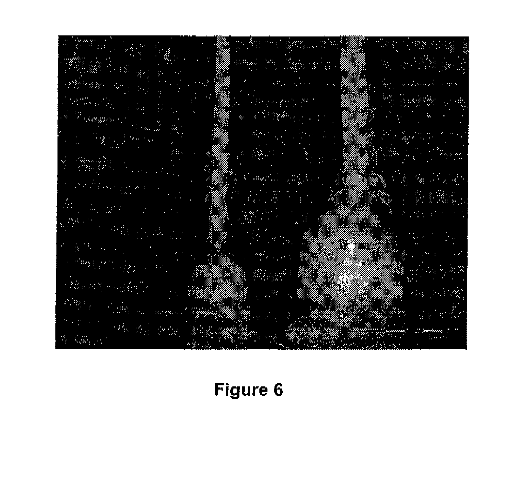

Figure 6 shows the result of the comparison between ovaries of day 0 (anthesis) of *END1::barnase* transgenic tomato plants and control plants. A: Ovary of a control flower in anthesis, B: Ovary of a *END1::barnase* flower in anthesis.

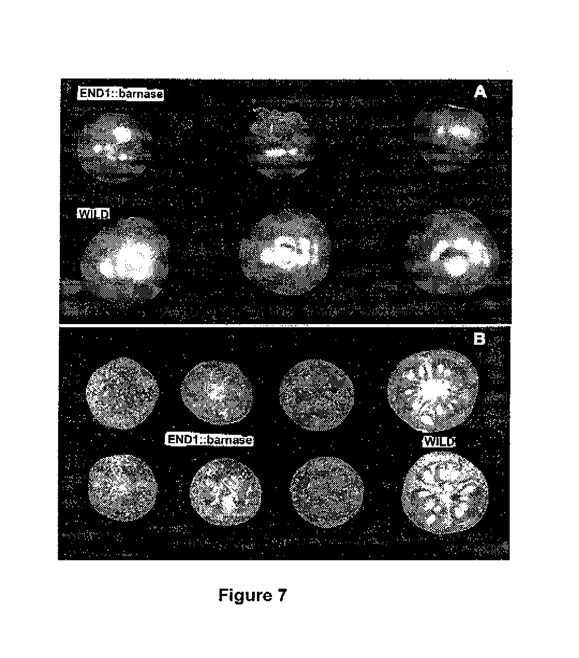

Figure 7 shows some parthenocarpic fruits developed by *END1::barnase* tomato plants (cv. Micro-Tom). A: Whole fruits of non-transformed plants and *END1::barnase* plants. B: Transversely sectioned fruits of non-transformed plants and *END1::barnase* plants.

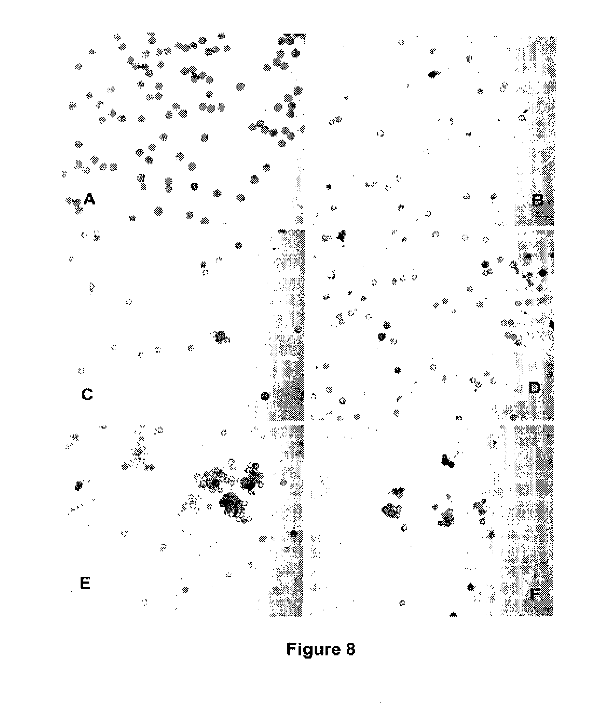

Figure 8 shows the result of vital staining with carmine acetate in pollen grains of *END1::barnase* tomato plants (cv. Micro-Tom). A: Pollen grains of the anther of a non-transformed plant. B: Pollen grains of the plant *END1::barnase* 14C. C: Pollen grains of the plant *END1::barnase* 2E. D: Pollen grains of the plant *END1::barnase* 12B. E: Pollen grains of the plant *END1::barnase* 15F. F: Pollen grains of the plant *END1::barnase* 4S.

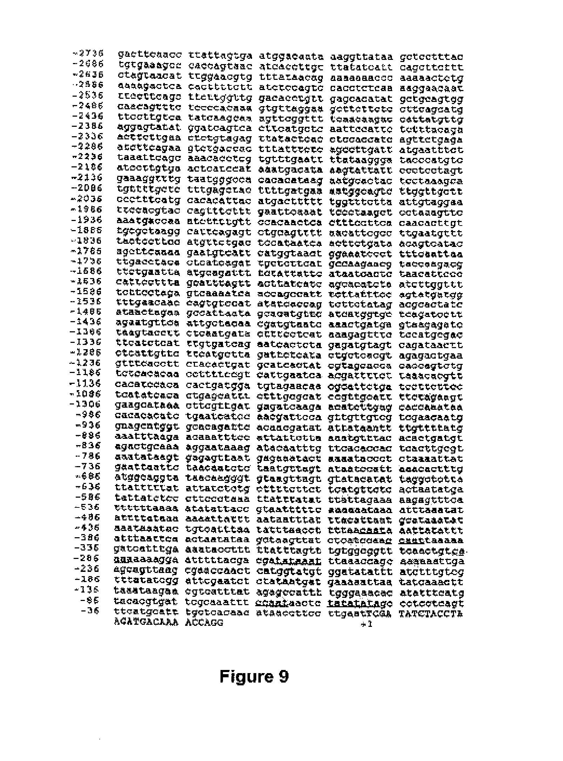

Figure 9 shows the sequence of the promoter region of the pea *END1* gene. The annotation +1 indicates the initiation codon (ATG).

### DETAILED DESCRIPTION OF THE INVENTION

In one aspect, the invention relates to a method for producing a transgenic tomato plant which produces parthenocarpic tomatoes, hereinafter the inventive method, which comprises the stages of:
(a) introducing a DNA construction into a cell or tissue of a tomato plant, said construction comprising:
   (i) the *END1* gene promoter of pea (*Pisum sativum* L.) or a fragment of that promoter capable of regulating the specific expression in anther; and
   (ii) a cytotoxic gene which is functionally bound to the aforementioned promoter or fragment of same,
      in order to produce a transformed cell or tissue of a tomato plant; and
(b) regenerating the tomato plant cell or tissue transformed in step (a) in order to produce a transgenic tomato plant;
in which said transgenic tomato plant produces parthenocarpic tomatoes when grown under conditions permitting the flowering and development of the tomato.

The term "transgenic tomato plant", as used in this description, refers to a plant which has been genetically manipulated in order to contain and express a heterologous DNA. In this invention, a transgenic tomato plant is genetically manipulated in order to stably and consistently contain and express a parthenocarpic phenotype not normally present in wild type plants. Likewise, the term transgenic tomato plant includes the progeny of the initial transgenic tomato plant which carries and is capable of expressing said parthenocarpic phenotype in its fruits.

"Parthenocarpic tomato", as used here, refers to a tomato (fruit) obtained in the absence of germination of the ovary, which completely prevents the formation of seeds and, therefore is entirely lacking in seeds. The transgenic parthenocarpic tomatoes provided by the present invention are somewhat smaller in size than wild ones and, in the mature state, display a more intense red colour than the corresponding wild ones (Figure 7).

The inventive method comprises the preparation of a DNA construction which consists of (i) the *END1* gene promoter of pea (*Pisum sativum* L.) or a fragment of that promoter capable of regulating the specific expression in anther; and (ii) a cytotoxic gene which is functionally bound to the aforementioned promoter or fragment thereof.

The *END1* gene promoter of pea *(Pisum sativum* L.) is a promoter capable of directing the specific expression in anther in early stages of the development of the plant as described and highlighted in patent application WO 01/073088. In fact, *in situ* hybridisation assays in that patent application confirmed the specificity of the expression of the *END1* gene in the tissues of the anther of pea, in particular, in the tissues comprising the pollen sacs of the anthers, during different stages of their development. Its expression started in very early stages of development (differentiation of common primordia in petals and stamens) and continued up to dehiscence of the anther, being expressed exclusively in the epidermis, connective tissue, intermediate layer and endothecium. No expression of the gene was detected, neither in the nutritive tissue (tapetum) nor in the germinal tissue (pollen). Similarly, the assays conducted with other floral organs, other parts of the plant (stem, leaves, roots, etc.) or with seeds (cotyledons) turned out to be negative. Therefore, the use of the promoter permits transgenic plants to be produced which express an anther specific gene.

The complete sequence of p*END1* nucleotides is shown in that patent application WO 01/073088 (SEQ ID NO: 1), and also in Figure 9 attached to this description.

In a particular embodiment, the p*END1* present in the DNA construction comprises the shown nucleotide sequence from nucleotide -2736 up to nucleotide -1 of the nucleotide sequence shown in Figure 9, which constitutes the complete sequence of that promoter.

In another particular embodiment, the DNA construction used for transforming cells or tissues of tomato plants consists of a p*END1* fragment which contains the nucleotide sequence from nucleotide -2736 up to nucleotide -6 of the nucleotide sequence shown in Figure 9. Said *pEND1* fragment maintains the regulating capacity of the specific expression in anther and is capable of directing the specific expression of anther in early stages of the development of the tomato plant, as revealed by the Examples accompanying this description.

In another particular embodiment, the DNA construction used for transforming cells or tissues of tomato plants consists of a p*END1* fragment which contains at least the nucleotide sequence from nucleotide -336 up to nucleotide -1 of the nucleotide sequence shown in Figure 9. As in the previous case, the previously defined *pEND1* fragment maintains the regulating capacity of the specific expression in anther and is capable of directing the specific expression of anther in early stages of the development of the tomato plant.

The *pEND1* can be obtained by conventional methods starting from a pea plant *(Pisum* sativum L.) or starting from a host organism transformed with a DNA sequence that includes that promoter, as mentioned in WO 01/073088. Likewise, the *pEND1* fragments which maintain the regulating capacity of the specific expression in anther can, on the basis of the information provided, be obtained by conventional methods, for example, starting from p*END1*, carrying out the appropriate deletions. In order to check if a p*END1* fragment maintains the regulating capacity of the specific expression in anther, the assays described in WO 01/073088 can be used (Example 1).

The DNA construction used for transforming cells or tissues of tomato plants includes, in addition to p*ENDl* or a functional fragment thereof, in other words, capable of regulating the specific expression in anther, a cytotoxic gene, functionally bound to said promoter or functional fragment thereof.

As used in this description, the term "cytotoxic gene" includes any gene which codes a protein that causes cell death in the tissue where, for example, a gene is expressed which codes a protein or enzymatic activity provoking the ablation of the anther. In plants, various proteins have been used that produce cell death, for example, diphtheria toxin A (DTA), naturally produced by *Corynebacterium diphteriae,* exotoxin A of *Pseudomonas* aeruginosa, ribonuclease T of *Aspergillus oryzae,* barnase of *Bacillus amyloliquefaciens,* etc.

In a particular embodiment, said cytotoxic gene expressed in anther due to the fact that it is under the control of p*END1* is the gene for barnase, a ribonuclease of *Bacillus amyloliquefaciens* [Mariani C, DeBeuckeleer M, Truettner J, Leemans J, Goldberg RB (1990) Induction of male sterility in plants by a chimaeric ribonuclease gene. Nature 347: 737-741], which provokes the complete ablation of the anther, from very early stages of its development, preventing the formation of pollen in it, and giving rise to an androsterile plant. This gene is particularly interesting because it has a powerful specific inhibitor of the ribonuclease activity of barnase, specifically the protein barstar, which can be used to restore fertility in androsterile lines [Mariani C, et al., (1992) Nature 357: 384-387] in order to obtain androfertile plants.

In a particular embodiment, the cytotoxic gene present in the DNA construction used for transforming cells or tissues of tomato plants is a gene which provokes androsterility in plants and said DNA construction comprises, in addition to (i) *pEND1* or a functional fragment thereof, and (ii) said gene which provokes androsterility in plants, (iii) a gene which reverts that sterility. By way of illustration, said gene which provokes androsterility in plants can be the barnase gene and said gene which reverts that sterility is the gene which codes barstar and those genes can be fused [Hartley, R.W. (1989) Barnase and Barstar: two small proteins to fold and fit together. Trends Biochem. Sci. 4, 450-454].

Additional examples of cytotoxic genes are cited in European patent application EP 412006 and also in patent application WO 01/073088, the contents of which are incorporated by reference to the present description.

The DNA construction used for transforming cells or tissues of tomato plants according to the inventive method can be obtained by conventional methods using widely known techniques [Sambrook et al., "Molecular cloning, a Laboratory Manual", 2nd ed., Cold Spring Harbor Laboratory Press N.Y., 1989 Vol 1-3]. Said DNA construction can also contain, functionally bound, certain regulating elements of the expression, for example, termination sequences of the transcription, booster sequences of the transcription and/or translation, etc.

The DNA construction used for transforming cells or tissues of tomato plants according to the inventive method can be inserted in the genome of a cell or tissue of a tomato plant by any suitable method for obtaining transformed cells and tissues of tomato plants. Said methods can involve, for example, the use of liposomes, electroporation, diffusion, particle bombardment, microinjection, gene guns, chemical compounds which increase the capture of free DNA, for example, coprecipitation with calcium phosphate, viral vectors, etc. Suitable vectors for the transformation of plants include those derived from the Ti plasmid of Agrobacterium tumefaciens, such as those described in EP 120516. In addition, with the transformation vectors derived from the Ti or Ri plasmids of *Agrobacterium,* alternative methods can be used for inverting the DNA construction in cells or tissues of tomato plants.

The DNA construction can be incorporated in a vector that includes a prokaryotic replicon, i.e., a DNA sequence capable of directing autonomous replication and maintaining the molecule of recombinant DNA extrachromosomically when introduced in a prokaryote host cell, such as a bacterium. Said replicons are known in the art. In a preferred embodiment, said prokaryotic replicon furthermore includes a gene whose expression confers a selective advantage, such as resistance to a drug (medicine), for the transformed host cell. Illustrative examples of bacterial genes which confer resistance to drugs include those which confer resistance to ampicilin, tetracycline, etc. The gene of the neomycin phosphotransferase has the advantage of being expressed in both eukaryotic and prokaryotic cells. Vectors which include a prokaryotic replicon also generally include some restriction sites for the insertion of the DNA construction used for putting the inventive method into practice. These vectors are known [US 6.268.552].

Among the expression vectors capable of expressing a sequence of recombinant DNA in plant cells and capable of directing the stable integration in the genome of the host plant cell are to be found vectors derived from the Ti plasmid of A. tumefaciens described by Rogers et al. [Rogers et al., (1987) Meth. in Enzymol. 153: 253-277] and various other systems of expression that are known which function in plants [see for example WO 87/00551; Cocking and Davey Science (1987) 236: 1259-1262].

In a preferred embodiment the expression vectors used in plant cells include an effective selection marker in eukaryotic cells, such as a selection marker for resistance to a drug. In a specific embodiment, the preferred marker for resistance to a drug is the gene whose expression confers resistance to kanamycin, in other words, a chimaeric gene which contains the promoter of the nopaline synthase, the gene of the neomycin phosphotransferase II and the terminator of the nopaline synthase, as shown in the examples accompanying this description.

The inventive method for obtaining transgenic tomato plants which produces parthenocarpic tomatoes comprises the introduction of the previously defined DNA construction into a cell or tissue of the tomato plant in order to produce a transformed cell or tissue of a tomato plant and generate a transgenic tomato plant by means of regeneration of said transformed cell or tissue of a tomato plant, wherein said transgenic tomato plant produces parthenocarpic tomatoes when grown under conditions that permit the flowering and development of the tomato.

The introduction of said DNA construction for transforming plant material and generating a transgenic plant can, as mentioned previously, be carried out by any means known in the state of the art, including though without being limiting to transfer of DNA mediated by A. *tumefaciens,* preferably with a disarmed T-DNA vector, electroporation, direct transfer of DNA, particle bombardment, etc. (see Davey et al., (1989) Plant Mol. Biol., 13:275; Walden and Schell (1990) Eur. J. Biochem. 192:563, Joersbo and Burnstedt (1991) Physiol. Plant. 81: 256; Potrykus (1991) Annu. Rev. Plant Physiol. Plant Mol. Biol. 42: 205, Gasser and Fraley (1989) Science 244: 1293; Leemans (1993) Bio/Technology, 11:522; Beck et al. (1993) Bio/Technology, 11:1524; Koziel et al. (1993) Bio/Technology, 11:194; Vasil et al. (1995) Bio/Technology, 11:1533).

The techniques for cultivating cells and tissues of transformed tomato plants and regenerating the transgenic tomato plants are well known [Ellul, P., Garcia-Sogo, B., Pineda, B., Rios, G., Roig., L.A, Moreno, V. (2003). The ploidy level of transgenic plants in Agrobacterium-mediated transformation of tomato cotyledons (Lycopersicon esculentum L. Mill.) is genotype and procedure dependent. Theor. Appl. Genet. 106, 231-238] as are the culture and growth conditions for the tomato plants with the aim of producing parthenocarpic tomatoes.

In addition, the systems and agents for introducing and selecting markers for checking the presence of heterologous DNA in plant cells and/or tissues are well known. Among the genetic markers which permit the selection of heterologous DNA in plant cells are to be found the genes which confer resistance to antibiotics, for example, kanamycin, hygromycin, gentamycin, etc. The marker permits the selection of satisfactorily transformed plants which grow in a medium containing the corresponding antibiotic because they carry the appropriate resistance gene.

Many of the useful techniques for carrying out the invention are conventional and known to technicians in plant biotechnology. By way of illustration, these conventional techniques are contained in Sambrook et al. (1989) Molecular Cloning, Second Edition, Cold Spring Harbor Laboratory Planview, N.Y.; Maniatis et al. (1982) Molecular Cloning, Second Edition, Cold Spring Harbor Laboratory Planview, N.Y.; Wu (ed.) (1993) Meth. Enzymol 218, Part I; Wu (ed.) (1979) Meth. Enzymol 68; Wu et al. (ed.) (1983) Meth. Enzymol 100 and 101; Grossman and Moldave (eds.) Meth. Enzymol 65; Miller (ed.) (1972) Experiments in Molecular Genetics, Cold Spring Harbor Laboratory, Cold Spring Harbor, N.Y.; Old and Primrose (1981) Principles of Gene Manipulation, University of California Press, Berkeley; Schleif and Wensink (1982) Practical Methods in Molecular Biology; Glover (ed.) (1985) DA Cloning Vol I. and II, IRL Press, Oxford, UK; Hames and Higgins (eds.) (1985) Nucleic Acid Hybridization, IRL Press, Oxford UK; Setlow and Hollaender (1979) Genetic Engineering: Principles and Methods, Vols 1-4, Plenum Press, New York, Kaufman (1987) in Genetic Engineering Principles and Methods, J. K. Setlow, ed., Plenum Press NY, pp. 155-198; Fitchen et al. (1993) Annu. Rev. Microbiol. 47: 739-764; Tolstochev et al. (1993) in Genomic Research in Molecular Medicine and Virology, Academic Press.

The present invention permits parthenocarpic tomatoes to be obtained without having to apply hormones (giberelines, auxines, cytoquinines) to unfertilised flowers, thus avoiding the need to have to use agrochemical products for the production of parthenocarpic fruits. An added advantage of the inventive method lies in the fact that pollination is not necessary in order to produce the fruit (tomato), thereby improving the efficiency of tomato production. As is known, poor pollination is one of the main causes of the production of incomplete and small size fruits, both in greenhouses and in the field.

The method provided by this invention implies the possibility of introducing the androsterility character, in dominant form, in any tomato cultivar suitable for genetic transformation. In fact, one of the main advantages of the method provided by this invention compared to others lies in its potential applicability to any tomato cultivar. The production of androsterile plants of pure lines of the cultivated varieties is fundamental for obtaining hybrids with greater productivity. Also, in cultivars for which interest in them is confined to using the vegetative parts, having androsterile plants implies being able to prevent undesired horizontal transfer of genes, which is especially relevant with regard to wild cultivars susceptible to suffering from cross-pollination, this being a transfer which implies one of the greatest concerns of ecological groups and of those citizens who are currently opposed to the culture of transgenic plants.

In another aspect, the invention relates to a method for producing a parthenocarpic tomato which comprises cultivating a transgenic tomato plant that produces parthenocarpic tomatoes obtained according to the inventive method described above, under conditions which permit flowering and development of the tomato. Said conditions are known to experts in the subject [Lifschitz, E., Brodai, L., Hareven, D., Hurwitz, C., Prihadash, A., Pnueli, L., Samach, A., Zamir, D. (1993). Molecular mapping of flower development in tomato. In Molecular Biology of Tomato, (ed.) J. Yoder, pp. 175-184. Technomic, Lancaster PA, USA. ISBN 0877629927; Ampomah-Dwamena, C., Morris, B.A., Sutherland, P., Veit, B., Y Yao, J-L. (2002). Down-Regulation of TM29, a tomato SEPALLATA homolog, causes parthenocarpic fruit development and floral reversion. Plant Physiol. 130 605-617].

The parthenocarpic tomatoes obtained according to the method stated above constitute an additional aspect of the present invention. Such tomatoes lack seeds, they are somewhat smaller than in the wild and, when ripe, they display a more intense red colour than the corresponding wild fruits (Figure 7). The parthenocarpic tomatoes provided by this invention can be used in foods and in the agrifood industries in the production of juices, sauces, pastas, purees, soups, etc., efficiently and economically without having to remove the seeds by sieving of the tomato pulp before processing it.

The following examples illustrate the invention and must not be considered as setting any limitation on it.

### EXAMPLE 1

### Functionality studies of pEND1 in transgenic plants of Arabidopsis thaliana, tobacco (Nicotiana tabacum) and tomato (Lycopersicon esculentum)

The specificity which the pea *END1* gene promoter confers for expression of foreign genes in the anther (WO 01/073088; and Gómez M.D.; Beltrán J.P.; Cañas L.A. 2004. The pea END1 promoter drives anther-specific gene expression in different plant species. Planta. In press (published online: DOI 10.1007/s00425-004-1300-z), offers the possibility of using it in cellular ablation assays of specific tissues thereof. The promoter would direct the expression of a cytotoxic gene in those tissues where *END1* is active and a check would be made of whether the destruction of the latter produced male sterility in those plants where that gene is expressed.

Previously (see example 1 of WO 01/073088), the inventors conducted functionality studies of that promoter in different species of pea by genetically transforming plants of Arabidopsis thaliana, *Nicotiana tabacum* and *Lycopersicon esculentum* by means of the specific expression in anthers of the marker gene uidA using the construction pBI101-F3 (Figure 1A). In order to check if p*END1* could be an efficient tool for the production of androsterile plants, they transformed those plants with the construction pBI101- *END1::barnase-barstar,* where the sequence of the promoter used [from the nucleotide (nt) - 2736 to nt -6 (WO 01/073088, Figure 2)] directed the specific expression of the cytotoxic gene of barnase to the anthers of those plants (Figure 1B). This cytotoxic agent was selected from among all possible ones owing to the fact that it has a powerful specific inhibitor of its activity (barstar) which can be used to restore the fertility in androsterile lines.

Likewise, the design was carried out of a construction pBI101- *END1::barstar* (Figure 1C) which carried the gene that codes for the specific inhibitor of barnase with the aim of restoring the fertility when used as a male parental line in the hybrids obtained by means of the controlled pollination of androsterile plants (female parental line).

The functionality of p*END1* in tomato was checked using the construction which carries the uidA gene (Figure 1A) under the control of that promoter. As can be seen in Figure 2 A-B, the expression of the GUS gene is specifically carried out in those tissues of the tomato anther that are involved in the architecture of the pollen sac (epidermis, endothecium, intermediate layer, connective) .

### EXAMPLE 2

### Obtaining of parthenocarpic tomato fruits (cv. Micro-Tom) associated with the production of androsterile plants by means of the specific ablation of anthers with a ribonuclease (barnase) controlled by the pea END1 promoter

### MATERIALS AND METHODS

### Plant material

Seeds of the Micro-Tom variety of tomato (*Lycopersicon esculentum*) were used as starting material.

### In vitro culture of tomato plants

The culture of tomato in glass flasks was carried out in order to germinate sterile seeds, so as to obtain the cotyledons which were going to be used as explants for the transformation and for carrying out the genotype analysis of the T₁ generation. This was conducted in cabins with a constant temperature of 25°C, under long-day photoperiod conditions (16 h light and 8 h darkness), with a light intensity of 90 E m⁻² s⁻² supplied by 36W Grolux type fluorescent tubes (Sylvania).

The tomato seeds were placed in mesh bags in a glass flask of diameter 9 cm and height 10 cm, containing 70% ethanol, for 1 minute and 30 seconds under constant stirring. They were then passed to a 30 g/l solution of bleach (starting from commercial bleach at 40 g Cl₂/l taking 75 ml thereof and bringing it up to 100 ml with sterile water), which also contained 0.02% of Tween^{®} 20 (SIGMA), which helps to break the surface tension between the tissues, improving contact between the tissue and the disinfecting agent. The disinfecting solution was then eliminated and 4 successive washes were performed with sterile distilled water (5, 10, 15, 20 minutes respectively). Finally, the water was eliminated and the bags were left with seeds inside the actual recipient.

Following disinfection of the seeds, the bags containing them were opened with some sterile tweezers, and they were taken one by one for being deposited on the surface of the germination medium which was contained in a glass jar of diameter 9 cm and height 10 cm. The composition of the germination medium was 15 g/l of saccharose, 2.2 g/l of MS salts [MS = Murashige and Skoog Medium (DUCHEFA)], 0.1 g/l of MES (4-morpholine-ethanesulphonic acid) pH 5.9, 3 g/l of agar. When the sterilised seeds were destined for genotype analysis, the germination medium also contained 50 mg/l kanamycin.

The tomato plants coming from in vitro culture were extracted from the flask and the roots were washed in order to eliminate remains of agar, trying not to damage them. They were then transplanted into plastic pots of diameter 13 cm which contained a mixture of peat:vermiculite (1:1) previously sterilised and acclimatised in a greenhouse cabin under controlled conditions, with a temperature of 24°C by day and 18°C by night. The natural light was supplemented with artificial light by means of mercury vapour lamps of 400 W [Philips HDK/ 400 HPI ^{®}, N], in order to maintain a long-day photoperiod. The watering consisted of a solution of Hoagland No. 1 supplemented with trace elements [Hewitt, Y. M. (1966). Sand and water culture methods used in the study of plant nutrition. Farnham Royal, Bucks. Commonwealth Agricultural Bureaux] contributed by means of an automated drip irrigation system for 2 minutes, 4 times a day.

### Cross-pollination

Cross-pollination was carried out in sterile male tomato plants in order to maintain the lines which, on their own, could not reproduce due to being androsterile.

The procedure consisted of directly applying pollen from the donor flower (stage at which the anthers are dehiscent) to the stigma of the carpel of the receptor flower (mother).

In the case of *END1::barnase* plants with severe malformation phenotype in the tomato anthers, it was not necessary to emasculate the receptor flower prior to anthesis due to the male sterility displayed by them. This was not the case for *END1::barnase* plants with medium and mild malformation phenotype of the tomato anthers, for which it was necessary to emasculate the receptor flower prior to pollination.

### Tomato genomic DNA extraction

In order to confirm the presence of foreign genes in plants assumed to be transgenic, molecular analysis was used based on the technique of polymerase chain reaction (PCR). To do this, genomic DNA needs to be extracted for being used as mould DNA in the PCR reaction.

The extraction of genomic DNA was done using the protocol of Rogers and Bendich (1984) [Rogers, S.O.; and Bendich, A. J. (1984). Extraction of DNA from milligram amounts of fresh, herbarium and mummified plant tissues. Plant Mol. Biology, 5, 69-76]. Approximately 200 mg of green leaf tissue were frozen using liquid nitrogen and were crushed in an Eppendorf tube with a metallic plunger until a very fine powder was obtained. 300 l of CTABx2 buffer [2% (v/v) CTAB (N-Cetyl-N,N,N-Trimethylammonium Bromide), Tris-HCl 100 mM pH 8.0, ethylenediaminetetraacetic acid (EDTA) 20 mM, NaCl, 1.4 M, 1% PVP (polyvinvylpyrrolidine) (Mr 40,000)] were added, which had previously been warmed to 65°C so that it could be pipetted correctly. It was mixed by inversion of the tube and left to incubate at 65°C. Once this buffer had finished being added to the final sample, all the samples were kept in incubation for a further 10 minutes, after which they were left to cool for 5 minutes at ambient temperature. Once that time had passed, 300 l of isoamyl chloroform (24:1) was added, and was mixed and centrifuged for 10 minutes at 11,000 rpm in order to separate the phases. Approximately 300 l of supernatant was collected and 30 l of CTABx10 buffer [10% (v/v) CTAB, 0.7 M NaCl] were added, preheated to 65°C. It was mixed and 300 l of isoamyl chloroform (24:1) were again added, and the samples were centrifuged under the above conditions. To the supernatants obtained were added the same volume of precipitation buffer CTAB [1% (v/v) CTAB, Tris-HCl 50 mM pH 8.0, EDTA 10 mM] after which the precipitate could immediately be seen and was collected by means of centrifugation for 10 minutes at 11,000 rpm. The supernatants were discarded and the precipitate was resuspended in 0.3 ml of saline TE buffer (Tris-HCl 10 mM pH 8.0, EDTA 1 mM, NaCl 1 M). It was again precipitated with 0.5 ml of absolute ethanol and the precipitate containing the DNA was finally collected following centrifugation at 11,000 rpm for 10 minutes. It was precipitated with 80% ethanol and left to dry. The DNA of each sample was dissolved in 50 1 of TE x 0.1 (Tris-HC1 1 mM pH 8.0, EDTA 0.1 mM) and was stored at -20°C.

DNA concentration was estimated by means of electrophoresis in a 0.8% agar gel using the techniques and reagents that are habitual in DNA analysis [ Maniatis, T.; Fritsch, E.F and Sambrook, J. (1982) Molecular cloning: a laboratory manual. Cold Spring Harbor Laboratory Press, Cold Spring Harbor, N.Y.].

### Culture of microorganisms

The liquid cultures of *Escherichia* coli and Agrobacterium tumefaciens bacteria were incubated during an entire night at 37°C and 28°C respectively, with stirring at 200 rpm. The cultures of E. coli and A. *tumefaciens* in boxes with solid medium were incubated during an entire night in a stove at 37°C and 28°C respectively.

| **Bacterial strains used:** | | |
|---|---|---|
| **Strain** | **Reference/origin** | **Use** |
| DH5 (*E. coli*) | Hanahan (1983) | Transformation ot bacteria |
| LBA4404 (*A. tumefaciens*) | Hoakema et al. (1983) | Transformation ot tomato |

Hanahan, D. (1983). Studies of transformation of Escherichia coli J. Mol. 166, 557-560 .
Hoekema, A., Hirsch, P.R., Hooykaas, P.J.J. and Schilperoort, R.A. (1983), A binary plant vector strategy based on separation of vir and T region of the Agrobacterium tumefaciens tiplasmid Nature. 303. 179-180.

The medium used for growing the microorganisms was LB medium (Luria-Bertani-Medium): 1% triptone, 0.5% yeast extract, 1% NaCl, pH 7.0. When solid medium was used, it was solidified with the addition of 1.5% agar (Pronadisa).

### Isolation of plasmidic DNA of Agrobacterium tumefaciens

For small-scale preparations of plasmidic DNA of A. *tumefaciens* the method of alkaline lysis was used, described by Sambrook et al. [Sambrook, J. Fritsch, E.F., Maniatis, T. (1989) Molecular cloning. A laboratory manual. Cold Spring Harbor Laboratory Press, New York] with slight modifications. The starting point was a culture of 3 ml, grown overnight in LB liquid medium supplemented with 50 g/ l of kanamycin. The sediment of cells resulting from centrifuging the culture was resuspended in 100 l of solution I [alkaline lysis method described by Sambrook et al. (1989) cited supra] and was treated as described in Sambrook et al. (1989). To the supernatant resulting from the centrifugation to which the lysate obtained after adding solution III was subjected [alkaline lysis method described by Sambrook et al. (1989) cited supra] were added 900 l of absolute ethanol and it was incubated for 30 minutes at -80°C. After centrifuging at 12,000 rpm for 5 minutes at ambient temperature, the precipitate was washed with 70% ethanol, dried and resuspended in 25 l of TE (EDTA 1 mM, Tris-Hcil 10 mM pH 8).

The purity of the DNA preparation obtained by this procedure was not sufficiently high for conducting a restriction analysis of the plasmid. In order to solve this problem, an aliquot of 1 l of this DNA preparation was used for transforming E. coli. From one of the transformant clones of *E*. *coli* obtained in this way, a new preparation of plasmidic DNA was made which was used for the pertinent analyses.

### Transformation by electroporation

The preparation of cells competent for their transformation by means of electroporation was carried out according to the protocols described in the catalogue Pulse Controller, Accessory for bacterial and fungal electro-transformation (BioRad) in the case of E. coli and according to Wen-Jun and Forde (1989) [ Wen-jun, S. and Forde, B. G. (1989) Efficient transformation of Agrobacterium spp. by high voltage electroporation Nucleic Acid Res. 17. 4415] in the case of *A*. *tumefaciens.*

After defrosting in ice an aliquot of 40 l of competent cells prepared by means of successive washings of glycerol, 1 l of transformant vector was added. The mixture was introduced into a tank with a 0.1 cm separation between electrodes (BioRad), previously chilled in ice, and it was subjected to an electric pulse with a Gene Pulser ^{®} apparatus (BioRad). The electroporation conditions were 200 , 25 F and 1.8 kV, for *E*. *coli,* and 400, 25 F and 1.8 kV, for A. *tumefaciens.* Following the electric pulse 1 ml of LB was added and incubation was carried out for 1 hour at 37°C at 200 rpm for E. coli, and for 3 hours at 28°C and 200 rpm for A. tumefaciens.

### Selection of bacterial recombinants

The selection of bacterial recombinants was carried out by means of sowing the transformed bacterial cells on dishes with LB medium (Luria-Bertani medium) supplemented with the antibiotic to which the plasmid under study had been conferred resistance and, in the event that the plasmid permitted selection by colour, 40 l (25 mg/ml) of IPTG (Isopropyl- -D-thiogalactoside) and 25 l (20 mg/ml) of X-Gal were added to the solid culture medium.

The antibiotics used for selection of bacterial recombinants and the concentration at which they were used appear in the following table:

| **Antibiotics used and their concentrations** | |
|---|---|
| **Antibiotic** | **Concentration** |
| Ampicilin | 100 g/l for *E. coli* |
| Kanamycin | 25 g/l for *E. coli* |
| | 50 g/l for *A. tumefaciens* |

### Design of the pBI101-END1::barnase-barstar construction

In order to test whether the expression of the cytotoxic gene *barstar* in those tissues of the anther where *END1* is active was capable of producing androsterility in transgenic tomato plants, the construction pBI101-*END1::barnase-barstar* was made.

To do this, the construction pBI101-F3 (Figure 1A) was started with. This
construction contained the sequence of the promoter region of *END1* [from nt -2736 to nt -6 (Figure 9)] isolated from the tracking of the genomic gene library of pea, directing the expression of the gene *uidA* which codes the enzyme - glucuronidase (GUS). This gene was released with the restriction enzymes *Bam*H1 and SacI, and the fragment corresponding to the plasmid pBI101 plus the promoter of *EMD1* was extracted from the agar gel. The barnase-barstar fragment (Figure 1B) previously cloned in the site *Bam*H1 of the plasmid pBluescript KS (+) (Stratagene) was expanded using the oligos:
Ribo I (5' TAGGAT*CCC*GACCATGGCACAGGTTATC 3') and
Inhi II (5' *GCGAGCTC*TTAAGAAAGTTGATGGTGATG 3')
With the first (Ribo I) the cutting site for the enzyme BamHl of the original clone is maintained at the level of the ATG of the *barnase,* while the latter (Inhi II) creates a cutting site for SacI at the level of the stop codon of the gene *barstar.* The fragment product of the PCR reaction was linked to the vector pGEM-T Easy (Promega) and was later on released with the enzymes BamHl and *Sac*I. This insert was cloned in the site which created those same enzymes in the construction pBI101-*END1*, thus creating the construction pBI101-ENDI::barnase-barstar (Figure 1B).

Barnase is a very active ribonuclease and, in order to manipulate this gene in prokaryotic organisms, the gene *barstar*, which is a specific inhibitor of the ribonuclease action of barnase, has been included in the construction. This gene functions solely as a bacterial gene. The capacity of the prokaryotic ribosome to translate all the cistrons of a messenger RNA each time it finds an initiation codon means that in these organisms, in the event of accidental expression of the barnase, its specific inhibitor can be expressed in the same way, thereby counteracting its destructive action. Eukaryotic organisms differ in this characteristic of translation.

### Transformation of Lycopersicon esculentum (Micro-Tom) and analysis of the transgenic plants

The genetic transformation of plants of *Lycopersicon esculentum* (cv. Micro-Tom) was carried out using the protocol optimised by Ellul et al. [Ellul, P., García-Sogo, B., Pineda, B, Rios, G., Roig., L.A, Moreno, V. (2003). The ploidy level of transgenic plants in Agrobacterium-mediated transformation of tomato cotyledons (Lycopersicon esculentum L. Mill.) is genotype and procedure dependent. Theor. Appl. Genet. 106, 231-238].

Briefly, cotyledons were taken from 12-day sterile plants, cultivated in germination medium (MG), and they were transversely cut into two segments eliminating the ends in order to increase the infection surface. The cotyledon explants were transferred to Petri dishes (24 explants/dish), which contained pre-culture medium (MPC), where they remained for 48 hours in a dark stove at 28°C. Once that time had passed, the explants were submerged for 6-8 minutes in 30 ml of the bacterial culture and then dried with sterile filter paper in order to eliminate the excess bacteria. They were positioned with the underside of the cotyledon in contact with the co-culture medium (MCC). The explants were incubated with the *A. tumefaciens* for 48 hours in a dark stove at 26°C. Once the co-culture period had ended, the explants were transferred for 10-12 minutes into sterile glass jars containing 150 ml of washing medium (ML) plus cephotaxim (500 mg/l) which halted the growth of *A. tumefaciens*. Following the washing, the explants were dried on sterile filter paper and placed on Petri dishes of 15 x 90 mm, containing organogenic medium without selective pressure (IK 4.0/4.0 + cephotaxim 400 mg/l), where they remained for 48 hours in a culture chamber. At the end of that period, the explants were transferred to Petri dishes of 25 x 90 mm containing about 20 ml of organogenic induction selective medium and were sub-cultivated following three weeks in these conditions. The calluses formed were separated from the explants at approximately 7-8 weeks eliminating the friable zones, and they were sub-cultivated every 3 weeks in the organogenic induction medium IKZ 4.0/4.0/1.0 until individual shoots appeared which were separated from the callus and transferred to rooting medium (ME). Once rooted, these shoots developed vegetative structures which permitted new plants to be obtained by means of clonal propagation. The molecular analyses and ploidy analysis to primary transformants were conducted on these vegetative structures.

The mineral and vitamin solutions and culture means used are described below.

| **Mineral and vitamin solutions** | |
|---|---|
| **Macronutrients** | **(mg/l)** |
| NH₄NO₂ | 1,650 |
| KNO₃ | 1,900 |
| CaCl₂. 2H₂O | 440 |
| MgSO₄.7H₂O | 370 |
| KH₂PO₄ | 170 |
| | |

| **Micronutrients** | **(mg/l)** |
|---|---|
| KI | 0.83 |
| H₃BO₃ | 6.20 |
| MnSO₄.4H₂O | 22.3 |

| **Germination medium (MG)** | |
|---|---|
| **MG** | **(g/l)** |
| Mineral solution | 100% |
| Saccharose | 10 |
| Agar | 8 |

| **Pre-culture medium (1 litre) (MPC)** | |
|---|---|
| Mineral solution (MS) | 100% |
| Saccharose (g) | 30 |
| Myo-inositol (mg) | 100 |
| Vitamins SH (ml) | 10 |
| Indolacetic acid (AIA) (mg) | 4 |
| Kinetine (mg) | 4 |
| Agar (g) | 8 |

| **Co-culture medium (1 litre) (MCC)** | |
|---|---|
| Mineral solution (MS) | 100% |
| Saccharose (g) | 30 |
| Myo-inositol (mg) | 100 |
| Vitamins SH (ml) | 10 |
| Indolacetic acid (AIA) (mg) | 4 |
| Kinetine (mg) | 4 |
| Acetosyringon (M) | 200 |
| Agar (g) | 8 |

| **Washing medium (1 litre) (ML)** | |
|---|---|
| Mineral solution (MS) | 100% |
| Saccharose (g) | 20 |
| Myo-inositol (mg) | 100 |
| Cephotaxim (mg) | 600 |

| **Organogenesis induction medium (MIO) (1 litre)** | |
|---|---|
| **IK 4.0/4.0 (for pre-culture)** | |
| Mineral solution (MS) | 100% |
| Saccharose (g) | 30 |
| Myo-inositol (mg) | 100 |
| Vitamins SH (ml) | 10 |
| Indolacetic acid (AIA) (mg) | 4 |
| Kinetine (mg) | 4 |
| Agar (g) | 8 |
| | |

| **IKZ 4.0/4.0/1.0 (for culture)** | |
|---|---|
| Mineral solution (MS) | 100% |
| Saccharose (g) | 30 |
| Myo-inositol (mg) | 100 |
| Vitamins SH (ml) | 10 |
| Indolacetic acid (AIA) (mg) | 4 |
| Kinetine (mg) | 4 |
| Zeatine (mg) | 1 |
| Agar (g) | 8 |

| **Rooting medium (ME)** | |
|---|---|
| **ME** | **(g/l)** |
| Mineral solution (MS) | 100% |
| Saccharose | 20 |
| Myo-inositol | 0.1 |
| Thiamine-HCl | 0.001 |
| Indolacetic acid (AIA) (mg) | 0.0001 |
| Agar | 8 |

| | |
|---|---|
| Notes: 1. The pH of the medium is adjusted to 5.7 with KOH prior to adding the gelling agent (agar). 2. The culture means are sterilised by damp heat in an autoclave, at 115°C for 30 minutes. 3. The vitamins are dissolved in water, and concentrated stock solutions of 100 X are prepared, they are stored at -20°C and added to the medium prior to adjusting the pH. 4. The zeatine is added sterile by microfiltration in a flow chamber following the sterilisation of the culture medium, at the rate of 1 mg/l. | |

### Evaluation of the ploidy level in the T₁ generation of pEND1::barnase tomato plants (Micro-Tom)

The ploidy level was analysed by means of flow cytometry, using leaves coming from the primary transformants cultivated in antibiotic-free rooting medium in order to isolate the nuclei. The tissue of approximately 1 cm² was placed on a Petri dish of diameter 50 mm to which was added 200 1 of nuclei extraction buffer (Partec), and it was chopped up with a knife. Once chopped, the resulting suspension was passed through a 50

1 nylon mesh (Nybolt) and 800 1 of a dye solution were added containing 1 mg/l of DAPI (4,6-diamino-2-phenyl-indol), thus achieving the fluorescent staining of the DNA. The DNA of the isolated nuclei was measured using a Partec PAS-II flow cytometer, fitted with a mercury lamp. The result appears traced on a semilogarithmic scale, in which the histogram which extends from 2C to 32C is distributed along the abscissa. In order to calibrate it, the peak 2C of young leaves of diploid tomato plantules cultivated *in vitro* was used.

### Germination test of pollen in vitro

In order to carry out the germination test of the pollen grains of transgenic tomato plants (Micro-Tom), the procedure described below was conducted.

Briefly, the liquid germination medium for the pollen was prepared (saccharose 0.292 M, Ca(NO₃)₂ 1.27 mM, H₃BO₃ 1.62 mM, KNO₃ 1 mM, KH₂PO₄ 0.1 mM) . 0.5% agar was added and it was fused in the microwave. A pipette was used to distribute it uniformly on glass slides and, once solidified, each slide was sprinkled with the pollen grains corresponding to an anther of an extended tomato flower. This process was carried out in triplicate for each plant.

The slides were incubated in the dark at a temperature of 25°C for 2 hours, after which a count was made of the pollen grains in a phase contrast inverted microscope (Nikon Diaphot). A pollen grain was considered to have germinated when the size of its pollen tube was equal to or larger than the diameter of the grain. Owing to the variability in the concentration of pollen grains found on each slide, different counting techniques were used for each case.

In the case of slides which contained pollen grains of wild plants, the count was conducted selecting 10 random fields. The mean of the results obtained was calculated and it was multiplied by 12 and by 27, which are the number of transverse and longitudinal fields, respectively, of the area of the slide.

For slides which contained pollen grains of plants 9L, 12B and 4S, the count was done making 5 longitudinal passes. The mean of these results was multiplied by 12. In the case of plant 14C, the count was done on the entire slide owing the small amount of pollen that was found.

### Analysis of the viability of pollen grains and observation of the results by optical microscope

In order to check the viability of the pollen grains of *END1::barnase* tomato plants (cv. Micro-Tom), the method of fixing and staining of the chromosomes with a solution of carmine acetate was used. To do this, the anthers of each tomato flower were squeezed so that the cells would undergo a mechanical separation. The content of the anthers (pollen grains) was placed on a side and stained with a solution of carmine acetate. The carmine acetate solution was prepared by diluting 0.5 g of carmine in 45% glacial acetic acid and heating it to boiling point. It was then diluted 1:1 in 30% glycerol. The samples were observed and photographed with an Eclipse 600 Nikon optical microscope.

### RESULTS

### Transformation of Lycopersicon esculentum (cv. Micro-Tom) with the construction pBI101-END1::barnase-barstar

Tomato transformation with construction *END1::barnase* generated 72 primary transgenic shoots (T₁), of which 25 were selected. The selected plants were analysed by means of flow cytometry with the aim of evaluating the ploidy level. Of the 25 transformants, 24 were diploid and just the plant 7A turned out to be tetraploid (Table 1). Analysis by means of PCR of tissue samples of the diploid transformants permitted the presence of the transgene to be detected in all of them (Figure 3). 14 plants were selected in order to be transferred to the greenhouse and be able to analyse their phenotypic characteristics (Table 1) .

The transgenic plants showed a development similar to that of non-transformed plants grown under the same conditions. The flowers of the control plants, as with those of the *END1::barnase* plants, developed the characteristic organs of the tomato flower: its five sepals alternating with five petals and two carpels fused to form a pistil.

Nevertheless, while the five stamens of the control plants formed the characteristic staminal cone around the style (Figure 4A), the phenotype of the stamens of the transgenic plants varied from one plant to another. Transformants were observed with stamens very similar to wild stamens (Table 1; Figures 4B and 4C, mild phenotype), others showed a slight reduction in their size and a small separation between them (Table 1; Figures 4D and 4E, medium phenotype) and finally other plants showed the atrophied stamens which left the style exposed (Table 1; Figure 4F, severe phenotype).

The transgenic anthers of the mild phenotype contained pollen grains in their interior and this could be observed by making an incision in them and knocking on a slide.

**Table 1**

| **Genotype and phenotype characteristics of T₁ generation *END1::barnase* tomato plants** | | | | | |
|---|---|---|---|---|---|
| **Independent calluses** | **Plant in medium with kanamycin** | **Ploidy level** | **Barnase-barstar PCR** | **Greenhouse plants** | **Anther phenotype** |
| | 1 a | 2x | + | | |
| | 1 d | 2x | + | X | Severe |
| 1 | 1 e | 2x | + | X | Severe |
| | 1 f | 2x | + | X | Severe |
| 2 | 2 d | 2x | + | X | Mild |
| | 2 e | 2x | + | X | Mild |
| | 4 a | 2x | + | | |
| 4 | 4 p | 2x | + | X | Mild |
| | 4 s | 2x | + | X | Mild |
| 5 | 5 b | 2x | + | X | Medium |
| 7 | 7 a | 4x | - | - | |
| | 9 b | 2x | + | X | Medium |
| | 9 h | 2x | + | | |
| 9 | 9 g | 2x | + | | |
| | 9 l | 2x | + | X | Medium |
| 11 | 11 c | 2x | + | | |
| | 11 d | 2x | + | X | Severe |
| | 12 b | 2x | + | X | Medium |
| 12 | 12 c | 2x | + | | |
| | 12 d | 2x | + | | |
| | 14 c | 2x | + | X | Medium |
| 14 | 14 d | 2x | + | | |
| | 15 d | 2x | + | | |
| 15 | 15 e | 2x | + | X | Mild |
| | 15 f | 2x | + | | |

### Comparative study of the development of a wild tomato anther (cv. Micro-Tom) and an anther of severe ENV1::barnase phenotype

A comparative study was conducted between the development of a wild tomato anther (cv. Micro-Tom) and that of a severe phenotype transgenic anther. To do this, anthers at different stages of development were immersed in resin and paraffin and transverse sections were taken with the aim of observing the tissues affected by the action of the cytotoxic gene and determining the moment at which it starts to affect the development of those tissues.

Figure 5 shows five representative stages of the development of a wild tomato anther (cv. Micro-Tom) in comparison with the equivalent stages of transgenic anthers. The explanation for the stages of development of the wild tomato anther was carried out taking into account those defined by Koltunow et al., 1990 [Koltunow, A. M., Truettner, J., Cox., K. H., Wallroth, M., Goldberg, R. B. (1990). Different Temporal and Spatial Gene Expression Patterns Occur during Anther Development. Plant Cell. 2, 1201-1224] for the development of the tobacco anther and those described by Brukhin et al., 2003 [Brukhin, V., Hernould, M., Gonzáles, N., Chevalier, C., Mouras, A. (2003). Flower development schedule in tomato Lycopersicon esculentum cv. sweet cherry. Sex. Plant. Reprod. 15, 311-320] for the sweet cherry variety of tomato.

### Stage 1

Wild: The first stage analysed shows an anther primordium where the archesporial cells have already become differentiated in primary and sporogenous parietal cells. The connective tissue, epidermis and vascular bundle are starting to form. The anther starts to achieve its bilobed shape.

*END1::barnase.* For this first stage, the transgenic anther already displays an abnormal appearance. It shape is different from that acquired by a wild stamen in the equivalent stage. This morphology recalls that of a wild anther in a lower stage of development. The main differences are concentrated in the four corners of the primordium, where the future pollen sacs are not seen to be marked.

### Stage 2

Wild: The epidermis, connective tissue and vascular bundle have formed. The walls of the pollen sacs are forming and include the endothecium, the *tapetum* and the intermediate layer, and the sporogenous tissue has become differentiated.

*END1::barnase:* The cells forming the epidermis show differences with those corresponding to a wild anther: they are round and small. There are fewer cells within the connective tissue and those that are going to give rise to the tissues surrounding the pollen sac are also fewer in number and have a different arrangement. At this stage of development the wild anther is achieving its characteristic external form. Their four loculi where the pollen sacs are going to be established are starting to be seen and the cells which are going to give rise to the pollen grains are starting to become differentiated in its interior. This is not observed in the transgenic anther in the equivalent stage.

### Stage 3

Wild: The *tapetum* can be seen surrounding the tetrads of microspores, which are in turn surrounded by callus. The other tissues of the anther are completely differentiated.

*END1: :barnase:* the external morphology of the anther shows signs of malformation: the pollen sacs are arranged differently to those of a wild anther, closer to one another and in their interior the *tapetum* surrounds possible tetraspores covered with callus. This is perhaps due to the fact that the connective tissue has collapsed causing the microsporangia to converge at a point.

### Stage 4

Wild: The anther shows signs of having entered into dehiscence: the *tapetum* and cells adjacent to the stomium have degenerated, the connective tissue has disappeared from the region which divided the two pollen sacs in the same lobe, making the anther bilocular. Vacuolated microspores can be seen inside the pollen sacs.

*END1::barnase:* There continue to be differences in the external morphology: the connective tissue has collapsed and there are no signs of having entered into dehiscence. Instead of mature pollen grains, an amorphous structure can be seen inside the pollen sacs and it seems to be surrounded by *tapetum.*

### Stage 5

Wild: The anther has opened via the region of the stomium and the mature pollen grains are being released via the opening that has been produced.

*END1::barnase:* The anther has become bilocular but the pollen sacs are deformed and inside there appear amorphous structures rather than mature pollen grains. The size of the anther is much smaller and its external morphology continues to be different from that of a wild anther in an equivalent stage. Although there are some signs of having entered into dehiscence with the bilocular arrangement of the anther, this has not reached its completion in order to give rise to the complete degradation of the connective tissue via the region of the stomium and cause its opening.

### Parthenocarpic development of fruits in END1::barnase transgenic tomato plants

The results obtained so far in terms of fertility of *END1::barnase* plants of *Arabidopsis* and tobacco show that the malformation in the anthers due to the action of the cytotoxic gene barnase generates male sterile plants which never reach the point of forming fruit.

Nevertheless in tomato, all the *END1::barnase* plants produce fruits. All the fruits produced lacked seeds and were therefore parthenocarpic, independently of the severity of the phenotype in the anthers and the presence of pollen grains observed for some transformants of mild phenotype. The development of the fruits in transgenic plants began earlier than in wild plants. The transgenic ovaries of flowers of day 0 (anthesis) reached a larger size than the wild flowers in the same stage of development (Figure 6).

The transgenic fruits were smaller and had a more intense red colour than that displayed by wild fruits (Figure 7). When these plants were emasculated and pollinated with pollen from anthers of non-transformed plants, fruits were obtained which achieved a normal size and which had seeds similar in number and morphology to those produced by wild plants. Data referring to differences in size and weight between parthenocarpic fruits and fruits obtained by cross-pollination in 4 independent transgenic lines appear represented in Table 2.

**Table 2**

| **Differences between parthenocarpic fruits generated by *END1::barnase* plants and fruits obtained by cross-pollination of these same plants** | | | | |
|---|---|---|---|---|
| **Genotype** | **Parthenocarpic fruits** | | **Fruits obtained by pollination** | |
| | **Weight (g)** | **Diameter (cm)** | **Weight (g)** | **Diameter (cm)** |
| Wild | - | - | 10 | 2.9 |
| 1 e | 4.25 | 2.05 | 9.35 | 2.55 |
| 4 s | 3.60 | 2.00 | 9.76 | 2.76 |
| 11 c | 6.61 | 2.33 | 9.48 | 2.88 |
| 12 b | 7.04 | 2.70 | 9.25 | 2.60 |

| | | | | |
|---|---|---|---|---|
| The data on weight and size of fruits shown correspond to the measurement of 6 fruits per plant. | | | | |

### Analysis of the viability and germination of the pollen in END1::barnase anthers of medium and mild phenotype

The presence of parthenocarpic fruits in *END1::barnase* plants was observed in all the plants of the T₁ generation, independently of the degree of malformation shown by the anthers and the presence of pollen grains. In order to check if these pollen grains were functional, the germination test was conducted on the pollen grains in vitro. A staining of them was also carried out with carmine acetate in order to observe their viability.

Table 3 shows that under the tested culture conditions a wild tomato anther (cv. Micro-Tom) had an average of approximately 18,000 pollen grains, of which 88% were capable of germinating. On the other hand, the anthers of *END1::barnase* plants that were analysed had between 10 and 100 times fewer pollen grains (between 28 and 972 grains/anther depending on the genotype) than wild anthers, and, of these, between just 4% and 8% of them germinated.

In Figure 8, which shows the staining of pollen grains with carmine acetate, it can be seen that the quantity of pollen grains of transgenic plants analysed is very much lower than the quantity of viable pollen grains in a wild anther.

In spite of noting the presence of a certain very small number of viable pollen grains and with germinative aptitude in anthers of *END1::barnase* plants, in none of the transgenic fruits was the presence of a single seed ever observed.

The reduction in quantity, viability and germinative capacity of the pollen grains of transgenic anthers, along with the morphological alterations of the stamens (style almost out of the staminal cone), are the factors probably responsible for the absence of seeds in the fruits of *END1::barnase* plants.

**Table 3**

| **Results of the *in vitr*o germination test of pollen grains of tomato (cv. Micro-Tom)** | | | | | | | | |
|---|---|---|---|---|---|---|---|---|
| | | Average No. of Pollen Grains / Anther | | | % | | | |
| Genotype | Sample *** | Germinated | Non-germinated | Total | Germinated | ES | Non-germinated | ES |
| | 1 | 19,213 | 3,110 | 22,324 | 86.1% | 0.002 | 13.9% | 0.002 |
| Wild | 2 | 14,645 | 2,236 | 16,880 | 86.8% | 0.003 | 13.2% | 0.003 |
| | 3 | 12,604 | 1,296 | 13,900 | 90.7% | 0.002 | 9.3% | 0.002 |
| | Average | **15,487** | **2,214** | **17,701** | **87.8%** | **0.189** | **12.2%** | **0.189** |
| 14 C | 1 | 0 | 5 | 5 | 0.0% | 0.000 | 100.0% | 0.000 |
| | 2 | 9 | 43 | 52 | 17.3% | 0.052 | 82.7% | 0.052 |
| | Average | **5** | **24** | **29** | **8.7%** | **0.162** | **91.3%** | **0.162** |
| | 1 | 2 | 34 | 36 | 6.7% | 0.042 | 93.3% | 0.042 |
| 9 L | 2 | 2 | 53 | 55 | 4.3% | 0.027 | 95.7% | 0.027 |
| | 3 | 0 | 55 | 55 | 0.0% | 0.000 | 100.0% | 0.009 |
| | Average | **2** | **47** | **49** | **3.7%** | **0.109** | **96.3%** | **0.109** |
| 12 B | 1 | 0 | 56 | 55 | 0.0% | 0.000 | 100.0% | 0.000 |
| | 2 | 12 | 96 | 108 | 11.1% | 0.030 | 88.9% | 0.030 |
| | 3 | 0 | 154 | 154 | 0.0% | 0.000 | 100.0% | 0.000 |
| | Average | **4** | **102** | **106** | **3.7%** | **0.109** | **96.3%** | **0.109** |
| | 1 | 88 | 916 | 1,004 | 8.8% | 0.009 | 91.2% | 0.009 |
| 4 S | 2 | 80 | 880 | 960 | 8.3% | 0.009 | 91.7% | 0.009 |
| | 3 | 76 | 876 | 962 | 8.0% | 0.009 | 92.0% | 0.009 |
| | Average | **81** | **891** | **972** | **8.4%** | **0.160** | **91.6%** | **0.160** |

| | | | | | | | | |
|---|---|---|---|---|---|---|---|---|
| ***, Each sample represents the slide where the count of the number of pollen grains was conducted. | | | | | | | | |

## Claims

1. A method for producing a transgenic tomato plant which produces parthenocarpic tomatoes, which comprises the stages of:
(a) introducing a DNA construction into a cell or tissue of a tomato plant, said construction comprising:
(i) the *END1.* gene promoter of pea (*Pisum sativum* L.) or a fragment of that promoter capable of regulating the specific expression in anther; and
(ii) a cytotoxic gene which is functionally bound to the aforementioned promoter or fragment of same,
in order to produce a transformed cell or tissue of a tomato plant; and
(b) regenerating the tomato plant cell or tissue transformed in step (a) in order to produce a transgenic tomato plant;
in which said transgenic tomato plant produces parthenocarpic tomatoes when grown under conditions permitting the flowering and development of the tomato.

2. Method according to claim 1, in which said pea *END1* gene promoter comprises the nucleotide sequence from nucleotide -2736 to nucleotide -1 of the nucleotide sequence shown in Figure 9.

3. Method according to claim 1, in which said DNA construction comprises a fragment of the pea *END1* gene promoter which has the nucleotide sequence from nucleotide -2736 up to nucleotide -6 of the nucleotide sequence shown in Figure 9.

4. Method according to claim 1, in which said DNA construction comprises a fragment of the pea *END1* gene promoter which comprises at least the nucleotide sequence from nucleotide -336 up to nucleotide -1 of the nucleotide sequence shown in Figure 9.

5. Method according to claim 1, in which said cytotoxic gene is a gene which provokes androsterility in plants when expressed in anthers.

6. Method according to claim 1 or 5, in which said cytotoxic gene is a gene which codes a ribonuclease activity or a gene which encodes a protein that causes cell death in the tissue where it is expressed.

7. Method according to claim 5, in which said cytotoxic gene is selected from among barnase gene, the gene which encodes diphtheria toxin A (DTA) naturally produced by Corynebacterium *diphteriae*, the gene which encodes exotoxin A of *Pseudomonas aeruginosa,* the gene which encodes ribonuclease T of *Aspergillus oryzae,* and the gene which encodes barnase of *Bacillus amyloliquefaciens*.

8. Method according to claim 1, in which said cytotoxic gene is a gene which provokes androsterility in plants and in which said DNA construction comprises, in addition to the (i) pea *END1* gene promoter or fragment thereof capable of regulating the anther specific expression, and (ii) said gene which provokes androsterility in plants, (iii) a gene which reverts that sterility.

9. Method according to claim 8, in which said gene which provokes androsterility in plants is the barnase gene and said gene which reverts that sterility is the gene which encodes barstar.

10. A method for producing a parthenocarpic tomato which comprises cultivating a transgenic tomato plant which produces parthenocarpic tomatoes obtained according to the method described in any of claims 1 to 9, under conditions which permit flowering and development of the tomato.

11. A parthenocarpic tomato, obtained by means of a method described in claim 10.

12. A parthenocarpic tomato **characterised in that** it lacks seeds, it is smaller than wild tomatoes and, when ripe, displays a more intense red colour than the corresponding wild tomatoes.
